# DEMANDE DE BREVET EUROPEEN

(11) **EP 1 106 198 A1**
(43) Date de publication de la demande: **13.06.2001**
(21) Numéro de dépôt: 00403298.3
(22) Date de dépôt: 24.11.2000
(51) Int. Cl.: A61M 16/01

(54) **Ventilateur d'anesthésie avec adjonction d'oxygène sans élévation notable de pression**

(30) Priorité: 02.12.1999 FR 9915212
(71) Demandeur: TAEMA, F-92182 Antony Cédex (FR)
(72) Inventeur: Masson, Jean-Philippe, 92370 Chaville (FR)
(74) Mandataire: Pittis, Olivier

(57) **Abrégé**

Appareil d'anesthésie respiratoire comportant un circuit principal (1) de gaz formé d'une branche inspiratoire (la) et d'une branche expiratoire (1b); un organe d'accumulation (5) de gaz communiquant avec le circuit principal (1) de gaz et contenant au moins un premier gaz; des moyens d'activation (16) actionnables par un utilisateur pour commander une adjonction d'un deuxième gaz, tel de l'oxygène, dans le circuit principal (1), et des moyens de pilotage (15) agissant, directement ou indirectement, sur l'organe d'accumulation (5) de gaz en réponse à un actionnement par l'utilisateur des moyens d'activation (16), pour empêcher ou limiter tout passage du premier gaz de l'organe d'accumulation (5) de gaz dans le circuit principal (5).

## Description

La présente invention se rapporte au domaine de l'anesthésie inhalatoire et, plus particulièrement, à un appareil d'anesthésie inhalatoire comprenant un circuit principal de gaz.

Les appareils d'anesthésie ou ventilateurs d'anesthésie comportent classiquement un système fermé de canalisations ou analogues, encore appelé circuit principal.

Ce circuit principal en boucle est destiné, d'une part, à amener jusqu'aux voies aériennes supérieures du patient des gaz anesthésiques frais issus d'une source de gaz anesthésiques frais et, d'autre part, de récupérer les gaz exhalés par le patient, de permettre une élimination du dioxyde de carbone contenu dans ces gaz exhalés par le patient, de permettre un recyclage et un mélange de ces gaz exhalés purifiés avec les gaz anesthésiques frais avant leur renvoi vers les voies aériennes supérieures du patient. Ce système à circuit fermé fonctionnant en boucle permet de minimiser la quantité de gaz anesthésique frais utilisée pour anesthésier le patient.

En général, le circuit principal de gaz se compose d'une branche inspiratoire amenant les gaz ver le patient et d'une branche expiratoire destinée à évacuer les gaz expirés par le patient avant leur purification et recyclage.

De tels dispositifs ont déjà été décrits à maintes reprises dans l'art antérieur.

Pour plus de détails, on peut se reporter notamment aux documents EP-A-643978, US-A-3687137, EP-A-292615 ou EP-A-266964.

Actuellement, les appareils d'anesthésie existants intègrent sur leur circuit principal un dispositif permettant de délivrer de l'oxygène pur à débit élevé au patient, lequel dispositif peut être, selon le cas, soit entièrement pneumatique, soit électronique.

En outre, un tel dispositif d'alimentation en oxygène pur est utilisable indifféremment en mode de ventilation spontanée, manuel ou mécanique.

Habituellement, la consommation en oxygène du patient dans le circuit principal est normalement compensée par l'apport en oxygène contenu dans les gaz frais.

Toutefois, un déséquilibre peut se produire en cas de modification physiologique du patient ou après une intervention de l'utilisateur sur le circuit principal.

L'envoi séquentiel au patient d'un débit rapide d'oxygène permet de compenser ou de rétablir l'équilibre plus rapidement.

Cependant, en mode de ventilation mécanique, l'adjonction de cet oxygène pur dans le circuit principal peut entraîner une augmentation importante de la pression interne de gaz régnant dans le circuit principal et donc aussi des voies aériennes supérieures du patient en engendrant alors un barotraumatisme de celui-ci, pouvant se traduire par des lésions pulmonaires diverses dues une augmentation brusque de la pression pulmonaire dudit patient.

Il se pcse donc habituellement un réel problème de sécurité pour le patient lors de l'adjonction d'oxygène dans le circuit principal, ce qui n'est pas acceptable compte tenu du risque potentiel pour le patient.

Pour tenter de pallier ce problème d'augmentation importante de la pression du circuit principal engendrée par l'envoi d'oxygène pur dans le circuit, il est d'usage d'isoler l'arrivée des gaz anesthésiques frais dans le circuit principal sous pression pendant l'envoi de l'oxygène pur ou, selon le cas, de limiter la pression régnant dans ledit circuit inspiratoire durant ladite adjonction d'oxygène pur en laissant échapper à l'atmosphère l'excédent de gaz contenu dans le circuit principal, via une valve de surpression ou analogue.

Or, ces techniques ont montré certaines limites.

En effet, dans le premier cas, l'isolement de l'apport de gaz frais implique que le patient ne peut pas bénéficier immédiatement de l'apport d'oxygène pur car la partie inspiratoire du circuit principal n'est alors pas balayée par l'oxygène d'apport.

En outre, il a été aussi constaté que la purge du circuit patient est alors aussi moins efficace.

Dans le second cas, le risque de barotraumatisme pour le patient n'est pas totalement éliminé car le patient est alors maintenu à la pression maximale préréglée au niveau de la valve d'échappement et ce, pendant toute la durée de l'administration de l'oxygène pur d'apport.

Dès lors, ces solutions connues ne peuvent pas être considérées comme pleinement satisfaisantes.

La présente invention se propose alors d'améliorer le niveau de sécurité pour le patient lors de l'activation de l'apport d'oxygène d'apport en proposant une solution technique n'engendrant pas d'augmentation notable de la pression dans le circuit principal de gaz et garantissant le maintien d'un balayage dudit circuit par l'oxygène d'apport, de manière à ce que l'apport d'oxygène puisse bénéficier immédiatement au patient et qu'un flux de gaz de purge soit maintenu.

La présente invention concerne alors un appareil d'anesthésie respiratoire comportant :
- un circuit principal de gaz formé d'au moins une branche inspiratoire et d'au moins une branche expiratoire,
- un organe d'accumulation de gaz communiquant avec ledit circuit principal de gaz et contenant au moins un premier gaz,
- des moyens d'activation actionnables par un utilisateur pour commander une adjonction d'un deuxième gaz dans le circuit principal, et
- des moyens de pilotage agissant, directement ou indirectement, sur l'organe d'accumulation de gaz en réponse à au moins un actionnement par l'utilisateur desdits moyens d'activation, pour empêcher ou limiter tout passage du premier gaz de l'organe d'accumulation de gaz au circuit principal.

Selon le cas, l'appareil selon l'invention peut comprendre l'une ou plusieurs des caractéristiques suivantes:
- l'organe d'accumulation de gaz comporte une paroi susceptible de se déformer sous l'action d'une pression externe ;
- l'organe d'accumulation de gaz est inséré dans une enceinte à pression interne variable ;
- les moyens de pilotage contrôlent la pression interne de ladite enceinte pour empêcher ou limiter ledit passage dudit premier gaz de l'organe d'accumulation de gaz au circuit principal ;
- des moyens à valve permettent de contrôler la pression interne régnant dans l'enceinte ;
- les moyens de pilotage agissent sur lesdits moyens à valve en réponse à un actionnement par l'utilisateur des moyens d'activation;
- les moyens de pilotage agissent sur lesdits moyens à valve pour permettre une diminution de la pression interne dans l'enceinte;
- des moyens d'échappement de gaz sont aménagés sur la branche expiratoire ;
- les moyens de pilotage agissent, en outre, sur lesdits moyens d'échappement de gaz en réponse à un actionnement par l'utilisateur des moyens d'actionnement;
- l'organe d'accumulation de gaz est un ballon-réservoir ;
- les moyens de pilotage comprennent au moins un microprocesseur et/ou un dispositif électronique éventuellement programmable permettant de piloter préférentiellement automatiquement les moyens d'échappement et/ou les valves ;
- les moyens d'actionnement comportent un moyen à bouton-poussoir.
l'invention porte aussi sur un appareil d'anesthésie, en particulier l'invention, comprenant un circuit principal de gaz formé d'au moins une branche inspiratoire et d'au moins une branche expiratoire, un organe d'accumulation de gaz communiquant avec ledit circuit principal de gaz, d'un dispositif d'administration d'oxygène relié au circuit principal par une ligne de connexion et commandé par un moyen d'actionnement manuel, d'un moyen d'échappement permettant de limiter la montée en pression du circuit principal à une pression proche de ou égale à la pression atmosphérique en réponse à l'activation du moyen d'actionnement.

L'invention concerne, en outre, un procédé de commande d'un appareil d'anesthésie, en particulier, selon l'invention, comprenant un circuit principal de gaz formé d'au moins une branche inspiratoire et d'au moins une branche expiratoire, un organe d'accumulation de gaz communiquant avec ledit circuit principal de gaz et contenant au moins un premier gaz, la paroi externe dudit organe d'accumulation de gaz étant susceptible de se déformer sous l'action d'une pression, dans lequel on envoie un gaz riche en oxygène dans le circuit principal et, sensiblement simultanément, on empêche ou on limite tout passage dudit premier gaz de l'organe d'accumulation de gaz au circuit principal.

Selon le cas, le procédé selon l'invention peut comprendre l'une ou plusieurs des caractéristiques suivantes:
- on empêche ou on limite tout passage dudit premier gaz de l'organe d'accumulation de gaz au circuit principal en diminuant la pression s'exerçant sur la paroi externe déformable de l'organe d'accumulation de gaz jusqu'à une pression-seuil inférieure ou égale à la pression du premier gaz contenu dans l'organe d'accumulation de gaz, de préférence jusqu'à une pression-seuil approximativement égale à la pression atmosphérique;
- le premier gaz est un gaz anesthésique et le gaz riche en oxygène est de l'oxygène sensiblement pur.

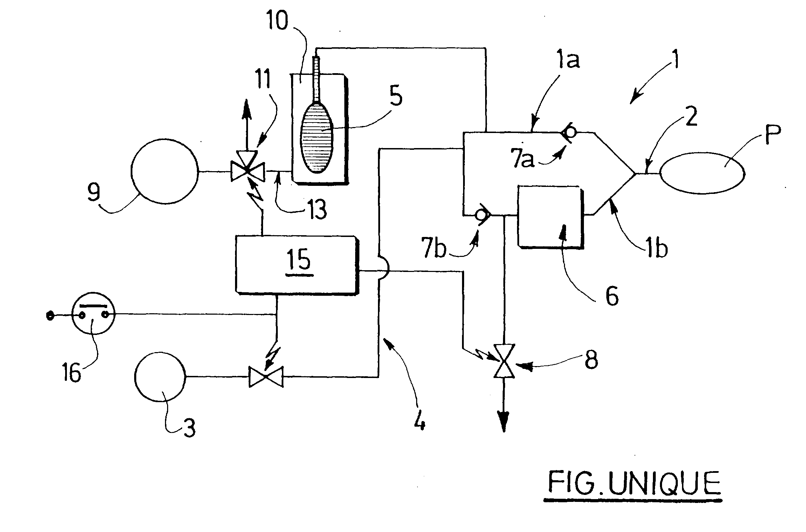

La présente invention va maintenant être décrite plus en détail en référence à la figure unique annexée, laquelle représente un appareil d'anesthésie inhalatoire conforme à la présente invention comprenant un circuit principal 1 d'anesthésie, se composant d'une branche d'inspiration 1a et d'une branche d'expiration 1b, lesdites branches 1a et 1b formant un circuit fermé ou circuit en boucle.

La partie aval de la branche inspiratoire 1a est reliée aux voies aériennes supérieures d'un patient (non représenté) par l'intermédiaire d'un moyen de raccordement 2 ou embout de raccordement, par exemple un masque respiratoire ou une sonde d'intubation, permettant de distribuer un gaz anesthésique aux voies respiratoires supérieures dudit patient.

En outre, la branche inspiratoire la est reliée par son extrémité amont, d'une part, à une source de gaz anesthésiques frais (non représentée), par exemple un mélange gazeux contenant de l'oxygène, de l'azote et des produits halogénés, et, d'autre part, à une source d'oxygène pur 3, via une ligne 4 d'alimentation du circuit principal 1 en ledit oxygène.

Par ailleurs, le circuit principal 1 comporte une branche expiratoire lb dont l'extrémité amont se raccorde à l'extrémité aval de la branche inspiratoire la au niveau du moyen de raccordement 2, de manière à recueillir les gaz expirés par le patient, et dont l'extrémité aval se raccorde à l'extrémité amont de la branche inspiratoire la à proximité d'un ballon-réservoir 5, de manière à constituer ledit circuit fermé.

La branche expiratoire lb comporte un dispositif 6 de purification permettant d'éliminer au moins une partie du dioxyde de carbone (CO₂) contenu dans les gaz expirés par le patient, par exemple une cartouche d'adsorbant.

La branche expiratoire lb comporte, en outre, des moyens à valve d'échappement 8 vers l'atmosphère destiné à pallier toute surpression dans le circuit principal 1.

En outre, des clapets anti-retour 7a et 7b sont aménagés sur lesdites branches inspiratoire la et expiratoire 1b, respectivement.

Durant le cycle de ventilation du patient du gaz anesthésique, les moyens à valve d'échappement 8 sont fermés pendant l'envoi de gaz au patient, c'est-à-dire pendant les phases inspiratoires du patient, pour permettre une montée en pression de l'ensemble du circuit 1.

A l'inverse, pendant les phases expiratoires du patient, les moyens à valve d'échappement 8 peuvent, si besoin est, s'ouvrir pour autoriser une sortie vers l'atmosphère de tout excès de gaz expirés et donc éviter toute surpression dans le circuit 1 principal.

Dans ce mode de réalisation, le ballon-réservoir, faisant office d'organe d'accumulation de gaz, est inséré dans une enceinte 10 pneumatique étanche.

Durant les phases inspiratoires, le ballon-réservoir est soumis à une compression par introduction d'un gaz-moteur, issu d'une source de gaz-moteur 9, à l'intérieur de ladite enceinte 10 de sorte que la pression du gaz-moteur s'exerçant sur la paroi externe du ballon-réservoir 5 entraîne une sortie du gaz anesthésique contenu dans les ballon-réservoirs 5, lequel gaz anesthésique peut ensuite être acheminé jusqu'au patient via la branche inspiratoire 1a.

A l'inverse, durant les phases expiratoires, le gaz-moteur contenu dans l'enceinte 10 est évacué, via une mise à l'atmosphère commandée par des moyens à valves 11, ce qui provoque une diminution de la pression s'exerçant sur la paroi du ballon réservoir 5 jusqu'à atteindre la pression atmosphérique. Le ballon-réservoir 5 peut alors être rempli par du gaz expiré par le patient et préalablement purifié (en 6), lequel est amené au ballon-réservoir 5 par la branche expiratoire 1b.

Selon la présente invention, le ventilateur comporte, par ailleurs, des moyens de pilotage 15 permettant de suspendre ou limiter l'envoi de gaz délivré par l'enceinte 10 de ventilation mécanique.

Plus précisément, les moyens de pilotage 15 agissant en réponse à un actionnement par l'utilisateur d'un moyen d'activation 16 manuel, tel un bouton-poussoir aménagé par exemple sur la face avant de l'appareil, pour limiter, et de préférence empêcher, toute sortie du gaz contenu dans le ballon-réservoir 5.

En fait, les moyens de pilotage 15, agissent sur les moyens à valve 11 pour autoriser une sortie du gaz-moteur contenu dans l'enceinte 10, lequel gaz-moteur est évacué à l'atmosphère.

En outre, les moyens de pilotage 15 commandent également la fermeture et l'ouverture des moyens 8 d'échappement à valve agencés sur la branche expiratoire lb pour assurer l'étanchéité du circuit principal 1 de gaz et l'acheminement du gaz enrichi en oxygène jusqu'au patient ou l'échappement à l'atmosphère du gaz en excès dans le circuit principal 1, en réponse au moyen d'activation 16 manuel.

En d'autres termes, lorsque l'opérateur, c'est-à-dire le praticien ou analogue, décide d'envoyer de l'oxygène pur au patient sous ventilation, il appuie sur le bouton-poussoir 16. L'actionnement du bouton-poussoir va, d'une part, entraîner l'envoi d'oxygène dans le circuit 1 principal et, d'autre part, être détecté par les moyens de pilotage 15, par exemple un microprocesseur, qui vont alors agir sur les moyens à valve 11 et les moyens d'échappement 8 pour mettre l'intérieur de l'enceinte 10 à la pression atmosphérique et empêcher ainsi toute sortie du gaz contenu à l'intérieur du ballon-réservoir, ce qui permet d'obtenir un échappement vers l'atmosphère des gaz du circuit principal 1 à une pression proche de la pression atmosphérique.

De cette manière, on évite toute surpression dans le circuit principal 1 et le patient n'est pas exposé au risque de barotraumatisme.

## Revendications

1. Appareil d'anesthésie respiratoire comportant :
- un circuit principal (1) de gaz formé d'au moins une branche inspiratoire (1a) et d'au moins une branche expiratoire (1b),
- un organe d'accumulation (5) de gaz communiquant avec ledit circuit principal (1) de gaz et contenant au moins un premier gaz,
- des moyens d'activation (16) actionnables par un utilisateur pour commander une adjonction d'un deuxième gaz dans le circuit principal (1), et
- des moyens de pilotage (15) agissant, directement ou indirectement, sur l'organe d'accumulation (5) de gaz en réponse à au moins un actionnement par l'utilisateur desdits moyens d'activation (16), pour empêcher ou limiter tout passage du premier gaz de l'organe d'accumulation (5) de gaz au circuit principal (1).

2. Appareil selon la revendication 1, caractérisé en ce que l'organe d'accumulation (5) de gaz comporte une paroi susceptible de se déformer sous l'action d'une pression externe, en ce que ledit organe d'accumulation (5) de gaz est inséré dans une enceinte (10) à pression interne variable et en ce que les moyens de pilotage (15) contrôlent la pression interne de ladite enceinte pour empêcher ou limiter ledit passage dudit premier gaz de l'organe d'accumulation (5) de gaz au circuit principal (1).

3. Appareil selon l'une des revendications 1 ou 2, caractérisé en ce que des moyens à valve (11) permettent de contrôler la pression interne régnant dans l'enceinte (10) et en ce que les moyens de pilotage (15) agissent sur lesdits moyens à valve (11) en réponse à un actionnement par l'utilisateur des moyens d'activation (16).

4. Appareil selon l'une des revendications 1 à 3, caractérisé en ce que les moyens de pilotage (15) agissent sur lesdits moyens à valve (11) pour permettre une diminution de la pression interne dans l'enceinte (10).

5. Appareil selon la revendication 1, caractérisé en ce que des moyens d'échappement (8) de gaz sont aménagés sur la branche expiratoire (1b) et en ce que les moyens de pilotage (15) agissent, en outre, sur lesdits moyens d'échappement (8) de gaz en réponse à un actionnement par l'utilisateur des moyens d'actionnement (16).

6. Appareil selon l'une des revendications 1 à 5, caractérisé en ce que l'organe d'accumulation (5) de gaz est un ballon-réservoir.

7. Appareil selon l'une des revendications 1 ou 5, caractérisé en ce que les moyens de pilotage (15) comprennent au moins un microprocesseur et/ou un système électronique permettant d'actionner les moyens à valve (11) et/ou les moyens d'échappement (8) en réponse à un actionnement des moyens d'actionnement.

8. Appareil selon l'une des revendications 1 à 7, caractérisé en ce que les moyens d'actionnement (16) comportent un moyen à bouton-poussoir.

9. Procédé de commande d'un appareil d'anesthésie, en particulier, selon l'une des revendications 1 à 8, comprenant un circuit principal (1) de gaz formé d'au moins une branche inspiratoire (la) et d'au moins une branche expiratoire (1b), un organe d'accumulation (5) de gaz communiquant avec ledit circuit principal (1) de gaz et contenant au moins un premier gaz, la paroi externe dudit organe d'accumulation (5) de gaz étant susceptible de se déformer sous l'action d'une pression, dans lequel on envoie un gaz riche en oxygène dans le circuit principal (1) et, sensiblement simultanément, on empêche ou on limite tout passage dudit premier gaz de l'organe d'accumulation (5) de gaz au circuit principal (1).

10. Procédé selon la revendication 9, caractérisé en ce qu'on empêche ou on limite tout passage dudit premier gaz de l'organe d'accumulation (5) de gaz au circuit principal (1) en diminuant la pression s'exerçant sur la paroi externe déformable de l'organe d'accumulation (5) de gaz jusqu'à une pression-seuil inférieure ou égale à la pression du premier gaz contenu dans l'organe d'accumulation (5) de gaz, de préférence jusqu'à une pression-seuil approximativement égale à la pression atmosphérique.

11. Procédé selon l'une des revendications 9 ou 10, caractérisé en ce que le premier gaz est un gaz anesthésique et en ce que le gaz riche en oxygène est de l'oxygène sensiblement pur.

12. Appareil d'anesthésie, en particulier, selon l'une des revendications 1 à 8, comprenant un circuit principal (1) de gaz formé d'au moins une branche inspiratoire (1a) et d'au moins une branche expiratoire (1b), un organe d'accumulation (5) de gaz communiquant avec ledit circuit principal (1) de gaz, d'un dispositif d'administration d'oxygène (3) relié au circuit principal (1) par une ligne de connexion (4) et commandé par un moyen d'actionnement (16) manuel, d'un moyen d'échappement (8) permettant de limiter la montée en pression du circuit principal (1) à une pression proche de ou égale à la pression atmosphérique en réponse à l'activation du moyen d'actionnement (16).
